# EUROPEAN PATENT SPECIFICATION

(11) **EP 1 046 038 B1**
(45) Date of publication and mention of the grant of the patent: **12.10.2005**
(21) Application number: 99900568.9
(22) Date of filing: 08.01.1999
(51) Int. Cl.: G01N 33/68, C07K 16/00

(54) **USE OF MASS FINGERPRINTING FOR IDENTIFICATION OF PROTEIN AFFINITY LIGANDS**
MASSENFINGERABDRUCKEN UND SEINER VERWENDUNG ZUR IDENTIFIZIERUNG VON PROTEIN-AFFINITÄTSLIGANDEN
UTILISATION DE LA CARTOGRAPHIE PEPTIDIQUE DE MASSE POUR L'IDENTIFICATION DE LIGANDS A AFFINITE PROTEIQUE

(30) Priority: 08.01.1998 GB 9800378
(43) Date of publication of application: 25.10.2000
(73) Proprietor: UNIVERSITY COLLEGE DUBLIN, Dublin 4 (IE)
(72) Inventor: PENNINGTON, Stephen, Roy, Chester CH3 7ER (GB)
(74) Representative: W.P. Thompson & Co.
(86) International application number: PCT/GB1999/000071
(87) International publication number: WO 1999/035502

(56) References cited:
- EP-A- 0 742 438
- WO-A-96/14580
- WO-A-98/15623
- M A KELLY ET AL: "Characterization of SH2-Ligand Interactions via Library Affinity Selection with Mass Spectrometric Detection" BIOCHEMISTRY, vol. 35, no. 36, 1996, pages 11747-11755, XP002081907
- P L COURCHESNE, S D PATTERSON: "Identification of Proteins by Matrix-Assisted Laser Desorption/Ionization Mass Spectroscopy Using Peptide and Fragment Ion Masses (from "2-D Proteome Analysis Protocols, Ed. A. J. Link)" METHODS IN MOLECULAR BIOLOGY, vol. 112, 1999, pages 487-511, XP002103063 cited in the application
- S R PENNINGTON, M R WILKINS, D F HOCHSTRASSER, M J DUNN: "Proteome analysis: from protein characterization to biological function" TRENDS IN CELL BIOLOGY, vol. 7, April 1997, pages 168-173, XP002103064 cited in the application

## Description

The present invention relates to the field of proteome analysis (proteomics) and to methods for the generation of tools for proteomics and the use of these tools in for example protein expression.

The last few years have heralded an unprecedented increase in gene sequencing and identification as exemplified by the announcement of the complete genome sequence of prokaryotic and eukaryotic cells and organisms including the model eukaryote *C. elegans* (see http://www.nih.gov/news/pr/dec98/nhgri-09.htm; Science 11 Dec 1998). The sequencing of the human genome continues to accelerate with completion projected for 3-4 years time. This growing DNA sequence data is being used as a platform for the systematic investigation of gene (mRNA) expression and function. Such investigations combined with associated informatics are transforming basic biological research and will transform the drug discovery, development and delivery processes. However, it has become increasingly apparent that DNA sequence data in itself often provides little information about the function of the encoded protein products. Furthermore, measurement of gene expression at the mRNA level does not always give an accurate representation of the expression of the corresponding proteins nor indeed of the extent to which they may be post-translationally modified. Importantly, it is predominantly proteins that execute biological function. Hence, there is a growing desire to analyse, in a systematic and comprehensive manner, the expression and activity of the protein products of an organism's genome - this aim provides a working definition of proteome analysis (or proteomics) (see Pennington *et al.* Proteome analysis: from protein characterisation to biological function. Trends Cell Biol. 7, 168-173 and references therein). It is important to emphasise that the activity of individual proteins may be regulated by a number of different mechanisms including their level of expression within individual tissues or cells, the extent and type of post-translational modifications, their subcellular localisation and their interactions with other proteins. Proteomics therefore encompasses a broad range of experimental approaches.

Existing approaches for proteomics are best exemplified by the use of two-dimensional electrophoresis (2-DE) to resolve and quantify the expression of several thousand proteins simultaneously with the application of a portfolio of methods to identify the resolved proteins. However, protein identification and full primary sequence determination still frequently rely on the application of existing DNA sequence data or of molecular biology based methods for identification of the corresponding gene sequence. Attempts are being made to develop alternative orthogonal approaches to high-throughput measurement of protein expression which, like 2-DE, are based on the initial separation of proteins from mixtures. It is notable however, that some of the most dramatic improvements in the measurement of mRNA expression have come from separation-independent methods that rely on *molecular recognition*. In summary, existing approaches for the measurement of protein expression have many inherent limitations - they are laborious, time consuming, suffer reproducibility problems, are not as sensitive as required and are unlikely to be readily applicable to the simultaneous analysis of all the proteins of even a 'simple' genome. Moreover, they have one overriding limitation - they do not result *per se* in the generation of experimental tools for measurement or manipulation of the proteins. So, for example, if 2-DE (or indeed any other existing protein based method) is used to identify a group of proteins associated with a particular disease (by differential analysis of protein expression) the process of identifying the proteins will not have led to the production of experimental tools. One example of such tools are molecules or ligands that can specifically bind to the individual proteins of interest with appropriate affinity for the desired application - 'protein affinity ligands'. Such tools have many uses. For example they might be used to measure protein expression, purify the protein for functional analysis, influence the activity of the proteins or be used as diagnostic reagents. The invention described herein provides a high throughput method to produce and identify such 'protein affinity ligands' either to proteins of interest or to proteins that may previously have been unidentified. It is an aim of the present invention to provide a high throughput method to produce 'catalogued' libraries of 'protein affinity ligands'. These 'protein affinity ligands' have many potential applications and will have applicability in drug target identification and validation, drug discovery and development and as diagnostic tools. They will also provide important tools for the development of separation independent approaches to the measurement of protein expression and post-translational modification.

The term 'protein affinity ligand' used herein refers to a molecule (large or small) that binds to a protein be it the polypeptide backbone (or part thereof) or any modified part of the protein, for example, a phosphorylated, fatty acylated, ADP-ribosylated or glycosylated moiety or indeed any known or hitherto unidentified modification either natural or introduced by experimental intervention. Such protein affinity ligands may be best exemplified by antibodies, polyclonal, monoclonal or phage-displayed, but include peptide or peptoid moieties (including peptide-nucleic acid molecules), oligonucleotides, modified oligonucleotides or indeed any molecule or chemical that binds to a protein with appropriate specificity and affinity. Chemicals might include those selected from combinatorial libraries. Hence in one embodiment, the invention is directed towards antibodies as the 'protein affinity ligands', although other affinity ligands may be produced and identified by the invention.

Described herein is a method, termed "inverse screening",of identifying and isolating 'protein affinity ligands' to individual proteins. Also described herein is a method of using mixtures of proteins to generate libraries of well characterised 'protein affinity ligands'. It is a feature of the invention that 'protein affinity ligands' to individual proteins may be generated without requiring access to the individual protein. The 'protein affinity ligands' so generated and identified by the present method and their protein targets (if previously unknown) are also therefore the subject of the present invention.

According to the present invention there is provided a method of selecting and / or identifying one or more protein affinity ligand's that bind to one or more proteins of interest comprising the steps of:
(A) obtaining a real or theoretical peptide mass fingerprint or other mass spectrometry based characterisation or other protein characterisation of the one or more proteins by either:
   i. Subjecting the one or more proteins to peptide mass fingerprinting or other mass spectrometry based characterisation or other protein characterisation; or
   ii. Predicting the peptide mass fingerprint or other mass spectrometry based characterisation or other protein characterisation from known data;
(B) utilising the one or more proteins either individually or as a mixture to:
   i. Generate one or more antibodies thereto by immunisation ; and/or
   ii. Select, using a single or multiple rounds of binding, one or more protein affinity ligands thereto;
(C) screening the one or more antibodies generated in step (B)(i) and/or the one or more protein affinity ligands selected in step (B)(ii) by:
   i. adding the one or more proteins individually or as a mixture of proteins to the one or more antibodies generated in step (B)(i) or the one or more protein affinity ligands selected in step (B)(ii), each antibody or protein affinity ligand being used individually, and
   ii. after removing any proteins which have not bound, eluting the at least one protein that has bound;
(D) subjecting the at least one eluted protein to peptide mass fingerprinting and / or other mass spectrometry based characterisation and/or other protein characterisation ; and
(E) by comparing the peptide mass fingerprints or other mass spectrometry based characterisation or other protein characterisation obtained in steps (A) and (D) selecting and/or identifying the at least one protein affinity ligand that binds to the one or more proteins of interest.

The other protein characteristics might include, for example, amino acid composition analysis or other sensitive and specific techniques.

The other mass spectrometry based characteristics might include, for example, the use of sequence tag data.

The mixture of proteins referred to above may be simple or complex; they may be obtained from any organism, any type of tissue, cell or combinations thereof that may have been subjected to any treatment. The protein mixture may also be obtained from fractionation of an organism, tissue or cell such as secreted, membrane, cytoplasmic, organellar or nuclear proteins or fractions of proteins bearing modifications for example carbohydrate containing or phosphorylated proteins. The protein mixture may also be obtained by *in vitro* expression of cDNAs. The proteins may be native or may have been modified in any way, for example, by biotinylation or by addition of fluorescent or other chemical moieties.

The 'protein affinity ligands' may specifically recognise more than one protein for example by way of binding to proteins that interact with each other. Under these circumstances the mass spectrometry data may be used to reveal such multiple proteins and the 'protein affinity ligands' may have additional value and/or use by virtue of their ability to recognise (i) multiple proteins of, for example, a protein family, or (ii) proteins that are bound to each other through protein:protein interactions.

Preferably the one or more proteins of interest are resolved by 2D electrophoresis.

Preferably the antibodies obtained in step (B)(i) are cloned prior to step (C).

Preferably the antibodies are immobilised on a solid phase support. Preferably the support is of nitrocellulose or PVDF. Such supports were found to be particularly effective at binding proteins in the methods of the invention.

Preferably the remaining binding sites on the solid support, after the antibodies are immobilised thereon, are blocked to prevent non-specific protein binding. Traditional agents proved ineffective for the methodology of the invention. However oligosaccharides and polyvinylpyrrolidines proved particularly effective.

Preferably a volatile reagent i.e one which is readily removed by for example evaporation, is used as the eluting agent. Formic acid was found to be particularly favoured since it could be used with a wide range of antibodies and was sufficiently volatile to be easily removed. Its use had the advantage that subsequent manipulation steps to remove the eluting agent were avoided.

In one embodiment of the present invention there is provided a method of generating monoclonal antibodies to one or more targeted proteins comprising the steps of:
(a) resolving individual proteins from a protein mixture;
(b) subjecting the resolved protein(s) to peptide mass fingerprinting to obtain a peptide mass profile;
(c) utilising one or more of the resolved proteins to generate one or more monoclonal antibodies thereto by immunisation and generation of hybridomas;
(d) adding a protein mixture to the one or more antibodies generated in Step (c) to select those proteins which bind the one or more monoclonal antibodies, and subjecting the selected protein(s) to peptide mass fingerprinting to obtain a peptide mass profile;
(e) comparing the data obtained in steps (b) and (d); and selecting one or more hybridoma clones of interest.

In this embodiment step (b) may be omitted where the protein(s) of interest are previously known and DNA sequence data for the corresponding gene(s) exits as under these circumstances step (e) could be undertaken by comparing the data obtained in step (d) with that obtained from theoretical peptide mass fingerprinting of the corresponding DNA sequence.

In a second embodiment of the present invention there is provided a method of generating a library of antibodies comprising the steps of:
(a) resolving individual proteins from a protein mixture;
(b) subjecting the resolved protein(s) to peptide mass finger printing to obtain a peptide mass profile;
(c) utilising a protein mixture to generate one or more monoclonal antibodies thereto by immunisation and generation of hybridomas;
(d) adding a protein mixture to the one or more monoclonal antibodies generated in Step (c) to select to those proteins which bind the one or more monoclonal antibodies, and subjecting the selected protein(s) to peptide mass fingerprinting to obtain a peptide mass profile;
(e) comparing the data obtained in steps (b) and (d); and,
(f) identifying the monoclonal antibodies of potential interest for a monoclonal antibody library.

In this embodiment steps (a) and (b) may be omitted where the protein mixture derives from an organism for which significant DNA sequence data is available as under these circumstances step (e) could be undertaken by comparing the data obtained in step (d) with that obtained from theoretical peptide mass fingerprinting of the potential open reading frames encoded in the DNA sequence data.

In a third embodiment of the present invention there is provided a process for selecting desired members of an affinity ligand library comprising the steps of:
(a) resolving a protein mixture;
(b) subjecting the resolved protein(s) to peptide finger printing to obtain a peptide mass profile;
(c) utilising one or more of the resolved proteins to select one or more affinity ligands from a library;
(d) adding the protein mixture to the one or more affinity ligands generated in step (c) to select those proteins which bind the one or more affinity ligands, and subjecting the selected protein(s) to peptide mass fingerprinting to obtain a peptide mass profile;
(e) comparing the data obtained in steps (b) and (d); and
(f) selecting one or more affinity ligands of interest.

In this embodiment steps (a) and (b) may be omitted where the protein mixture derives from an organism for which significant DNA sequence data is available as under these circumstances step (e) could be undertaken by comparing the data obtained in step (d) with that obtained from theoretical peptide mass fingerprinting of the potential open reading frames encoded in the DNA sequence data.

In each embodiment the protein mixture used in step (d) may or may not be the protein mixture used in the prior steps.

Hence, the process represents a novel linkage between the resolution and identification by a protein characterisation, for example, mass spectrometry of individual proteins present in protein mixtures with the screening of affinity ligands by such means. The most significant advantage of the process is the provision of a method to generate and screen affinity ligands to proteins without requiring pure protein. To date, no other screening process which circumvents the need for pure protein has been described. Furthermore, the present invention provides a method to generate large numbers of 'protein affinity ligands' for which the target proteins are identified without requiring access to the pure proteins.

The present invention includes provision for undertaking those steps in which proteins are selected by a 'protein affinity ligand' and subsequently analysed by, for example, mass spectrometry (i.e. step (d)) directly on the mass spectrometry targets or alternatively after some additional processing of'protein affinity ligands' or the selected proteins prior to mass spectrometry. The present invention also includes provision for isolation and manipulation of'protein affinity ligands', for example affinity isolation of antibodies from tissue culture supernatants, prior to use for inverse screening

A number of aspects of the invention are described in more detail, by way of example only, with reference to the following examples and flow diagrams.

### Comparative Example

### Current Approach

In a conventional approach to the generation of antibodies, an antigen or mixture of antigens is administered to mice, rats or other animal and the immune response to the antigen(s) is monitored by ELISA, dot blotting or, less frequently, Western blotting. Having identified animals which have produced an adequate immune response, monoclonal antibodies may be produced by taking the spleen cells from the animal and fusing them with cells of a myeloma cell line to generate hybridomas - immortalised cells which produce antibodies. The hybridoma cells are cloned, for example, by limiting dilution and the clones then screened by a range of different screening approaches to determine which cells are producing the desired antibodies. For effective selection of hybridomas the screening process must be robust, rapid and reliable because it is undertaken in parallel with the initial culture of the hybridoma cells. There are three classes of screening strategy: antibody capture assays, antigen capture assays and functional screens. In general, the vast majority of screens are currently undertaken by antibody capture. In this approach the pure antigen bound to a solid surface is used to capture the antibodies and these are then detected with appropriate labelled anti-immunoglobulin antibodies. This process screens for antibodies to an individual protein antigen and requires significant quantities of purified protein. Antigen capture screens are rarely used unless the protein antigen is available in large quantities - this is because they require labeling of the antigen (often with radioisotopes i.e. ¹²⁵I).

Thus, although immunisation may require only small amounts of an individual protein antigen (often less than 1 µg is appropriate) the screening process requires relatively large quantities of pure protein. Thus, in the context of current approaches to proteome analysis, where 2-DE is used to resolve thousands of proteins in sub-µg quantities existing methods for antibody production and isolation are unsuitable.

Another important feature of the existing methods is that those hybridoma clones or mixtures thereof that do not produce antibodies that recognise the target antigen are discarded. In the present invention the hybridomas or other 'protein affinity ligands' are cloned at an early stage and each antibody screened. By archiving the cloned hybridomas or other'protein affinity ligands' it is possible to re-screen them under different conditions and against different protein mixtures.

The process described below provides a novel approach to the widespread production of antibodies or other 'protein affinity ligands' to proteins individually or in mixtures. The novel linkage between resolution of protein mixtures and the screening of affinity ligands by, for example, mass spectrometry provides a powerful approach to the generation of characterised 'protein affinity ligands'.

### Example 1

### Process directed towards generation of monoclonal antibodies to 'targeted proteins'

A protein mixture is separated by, for example, two dimensional electrophoresis (2-DE) or by any other means to provide individual 'target proteins'.

The proteins of interest are subjected to peptide mass fingerprinting or other mass spectrometry based method - this gives a characteristic peptide pattern and other data such as sequence tag that are unique to each of the proteins and it is this data that is exploited to select antibodies which are capable of binding the 'target proteins'.

Antibodies secreted from individual hybridoma clones are allowed to interact with a protein mixture containing the protein or proteins for which antibodies are desired The appropriate antibodies will bind the target protein(s) of interest and the protein bound by each antibody may then be eluted and subjected to peptide mass fingerprinting or other mass spectrometry based analysis. The specificity and sensitivity of the mass spectrometry will allow the identification of the target protein(s) of interest and so reveal which hybridoma cells produce antibodies to the target protein(s).

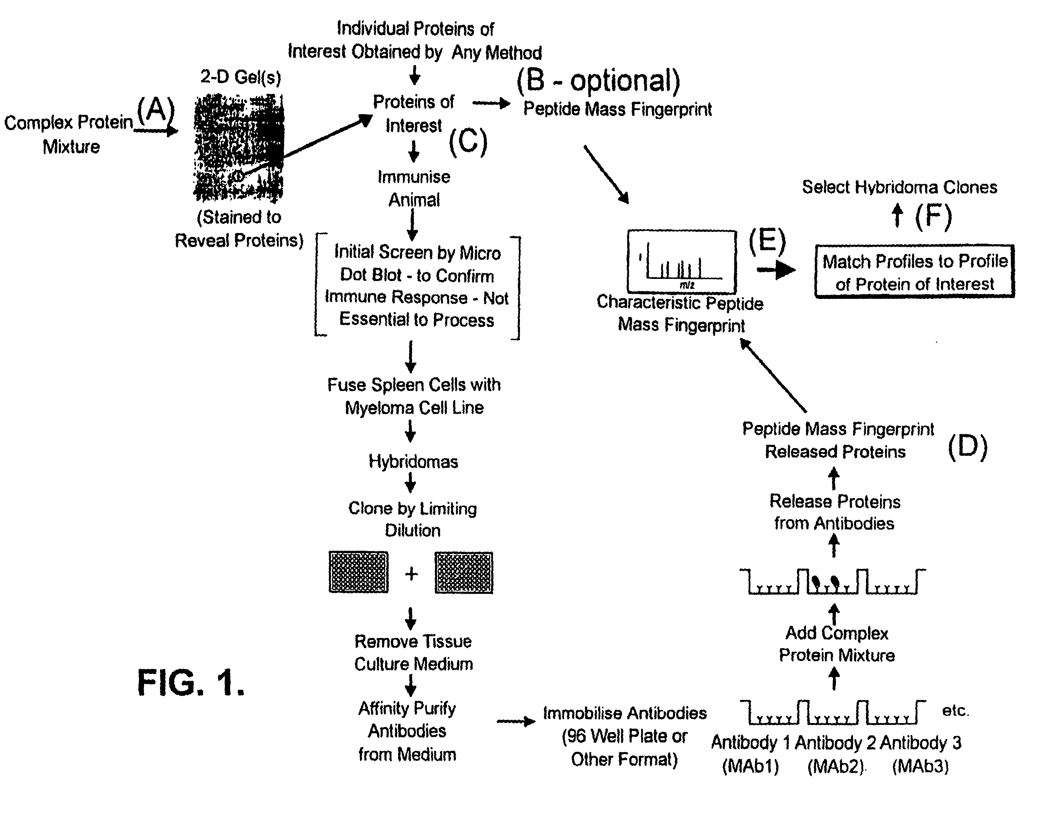

This method is described more fully with reference to Fig. 1, which is a flow diagram of the method.

To generate monoclonal antibodies to protein(s) of interest, mammals, for example, rats or mice are immunised (Step C) with individual proteins recovered from, for example, 2-DE gels. An aliquot of the protein(s) may be subjected to peptide mass fingerprinting (Step B).

After fusing spleen cells with myeloma cell lines the resulting hybridomas are cloned, for example, by limiting dilution of the cells. The antibodies produced by individual clones or mixtures of clones are screened by mass spectrometry. Thus, monoclonal antibodies are recovered from the tissue culture media and immobilised to a solid phase support which may for example be a 96 well plate. To the immobilised monoclonal antibodies is added a protein mixture and the proteins which have bound to individual monoclonal antibodies eluted and subjected to peptide mass fingerprinting (Step D) or other mass spectrometry based characterisation. By comparing the data (Step E) obtained with the data for the proteins of interest it will be possible to select (Step F) those monoclonal antibodies which are specific for the proteins and hence isolate the relevant clones.

In a further improvement the process is streamlined by application of automated multi-well plate handling technology and automated high-throughput mass spectrometry for example by use of automated sample target loading so facilitating the screening of large numbers of individual cell clones.

### Example 2

### Process directed towards the generation of an antibody library

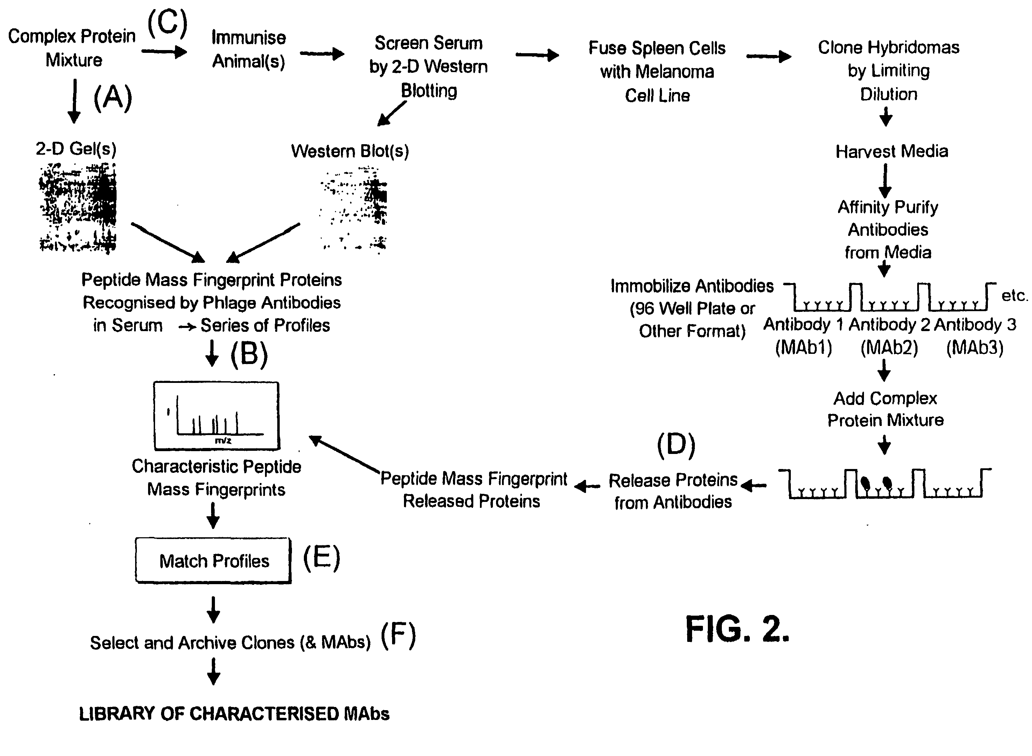

This process is described with reference to Fig. 2, which is a flow diagram of the method.

Mice/rats are immunised (Step C) with a mixture of proteins and the immune response may be screened by ELISA using the protein mixture. To establish the 'distribution' of antibodies generated - i.e. the coverage of the polyclonal library, the antiserum may be used for Western blotting for example, against 1-DE or 2-DE resolved proteins of the original protein mixture (Step A) or any other protein mixture. For those animals which produce antibodies of the desired coverage the spleen cells are fused with a myeloma cell line to generate hybridomas and the antibodies produced by the total pool of hybridoma cells may be re-screened by, for example, Western blotting against the 1-DE or 2-DE resolved proteins. This may be used to confirm that suitable antibody producing hybridomas have been generated. The hybridoma mixture is then cloned for example by limiting dilution and antibodies immobilised, a complex mixture of proteins added, the proteins which have bound eluted (Step D) and screened by mass spectrometry. As with example 1 the data can be compared (Step E) and the clones of interest selected (Step F).

The key advantages of the approaches outlined above are:
purified protein is not required for screening;
monoclonal cell lines are relatively stable and can be archived;
antibodies of high affinity and appropriate selectivity will be produced; and
the protein mixture used for screening the antibodies can be presented to the antibodies in a form tailored to the likely end application i.e. the proteins may be presented in native or denatured form.

### Example 3

### Process for selecting phage displayed antibodies

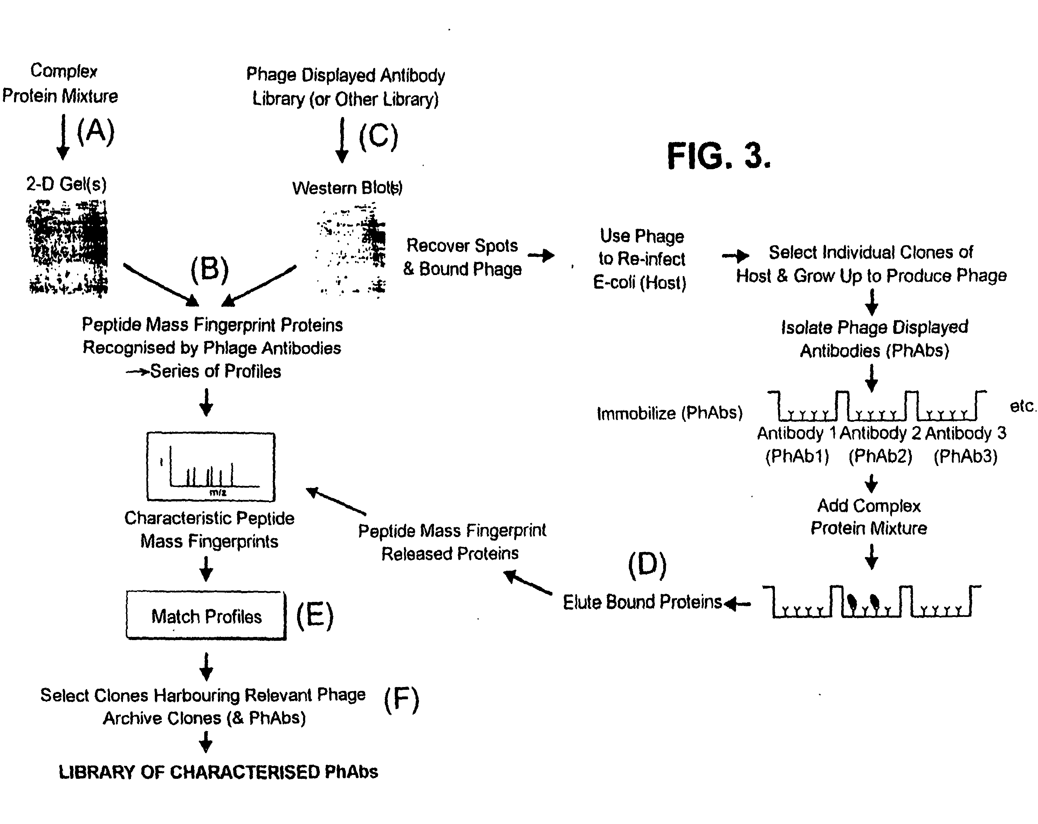

This process is described with reference to Fig. 3, which is a flow diagram of the method.

Following separation of protein mixtures by, for example, 2-DE (Step A), the proteins are Western blotted (Step C) using an antibody phage library with detection of bound phage. Individual protein spots of interest and associated antibody displaying phage are excised from the Western blot, the phage eluted and used to re-infect *E. coli.* Individual colonies of *E. coli* may be propagated (in 96 well plate format), phage antibody secretion induced with IPTG and individual antibodies (PhAbs) recovered. The PhAbs are then immobilised and a protein mixture added. Proteins which bind are then eluted (Step D) and characterised by peptide mass fingerprinting. As with Example 1 the data can be compared (Step E) and PhAbs which bind the protein(s) of interest will be selected (Step F).

The key advantages of the approach outlined above are:
it avoids the typical 4-5 rounds of phage selection but may also be used with any conventional phage selection process;
purified protein is not required for screening;
the method also reveals whether any PhAbs which bound to the protein during Western blotting are specific for other proteins in the protein mixture. Each screen will therefore produce PhAbs to at least the protein of interest and
potentially others as well;
the phage can be archived to generate a stable source of PhAbs (Step F). modification of the Western blotting selection conditions and the conditions under which protein mixture is added to and washed from individual PhAbs can be modified to isolate PhAbs of desired properties (i.e. affinity, avidity); and
relevant selected phage can be genetically modified to alter properties of PhAbs.

### Example 4

### Inverse screening a polyclonal antiserum to a 'target protein' - bovine serum albumin

The inverse screening method was used to screen a commercially available polyclonal antibody to bovine serum albumin (BSA) against a mixture of proteins (containing BSA). Polyclonal anti-BSA antibody (5-80µg; Sigma B-3759) in phosphate-buffered saline (PBS) was immobilised in the wells of 96 well filtration plates that incorporated a high protein binding hydrophobic PVDF membrane (Millipore MultiScreen® Immobilon™-P filtration plates) by incubating the pre-wetted membranes with the antibody containing solution for 2h at room temperature. The membranes were pre-wetted according to manufacturers instructions; thus to each well was added 50µl of 50% ethanol and the membranes incubated for 1 min at room temperature and after vacuum aspirating the wells each well was washed twice with 200µl of de-ionised water or PBS. After antibody binding the wells were washed extensively with PBS (6 x 0.35ml). It is clear that different conditions for the immobilisation of antibody may be used and many have been examined in detail. These other methods include use of different buffers, different incubation times and temperatures, different methods for removal of remaining antibody solutions (vacuum aspiration through membrane or direct removal) all of which are known to those of skill in the art. The method described here proved optimal for the antibody used in this example. Other methods described where appropriate were optimal for the other examples cited The use of conventional 96 well plates or other solid phase supports commonly used in immunoassays did not have sufficient protein binding capacity for the immobilisation of antibody. This is important because sufficient protein has to be recovered from the antibodies for subsequent characterisation.. The use of a solid phase support, in this case PVDF, in a suitable format for high throughput application represents a significant inventive step and is critical for some of the examples cited herein.

The extent to which antibody binding had been effected was examined by several methods. Most routinely it was examined by determining the protein concentration (Bradford assay) of the antibody solution after it had been incubated with the wells. Under the conditions described greater than 95% of the antibody was immobilised when antibody solutions containing up to 40µg of antibody were added to each well. The maximum protein binding recorded (using solutions containing up to 80µg of antibody) was 44µg which is in close agreement with the manufacturer's data.

It was reasoned that the ability to identify the proteins eluted from immobilised antibody would be critically dependent on the efficacy with which remaining protein binding sites on the solid phase support could be blocked to prevent subsequent non-specific protein binding. Thus, it seemed likely that non-specific proteins eluting with the antibody bound protein would significantly reduce the ability to obtain a peptide mass fingerprint suitable for matching. It was for this reason that a number of different blocking reagents and conditions were examined. Unfortunately, many of these reagents (detergents such as Tween and Triton, protein blocks etc.)were found to impair peptide mass fingerprinting by MALDI mass spectrometry. Consequently the applicant investigated a number of blocking reagents including polyvinylpyrrolidones and oligosaccharides that are not normally used in immunoassays. It was found that the quality of peptide mass fingerprinting data obtained from proteins eluted from antibody was significantly enhanced by use of these named blocking reagents, particularly ficoll. This represents an important inventive step. Thus, once antibody had been immobilised the remaining protein binding sites were blocked by incubation with 0.5% ficoll in PBS for 2h at room temperature. The wells were then washed extensively with PBS (6 x 0.35ml), incubated with elution reagent (0.5% formic acid) and washed again with PBS (6 x 0.35ml). A further method also found to be effective involved combining the ficoll block described above with dry blocking as recommended for the manufacturer for treatment of Immobilon™-P membranes when used in standard Western blotting applications. In this approach, the wells were dried by carefully removing all remaining solution and incubating in a desiccated environment at 37°C for 2h. The wells were then treated with 0.5% ficoll in PBS for 2h at room temperature, washed, treated with elution reagent and washed again as described above.

Having immobilised the antibody and blocked remaining protein binding sites on the solid phase support the antibody is exposed to a protein mixture and specifically bound protein(s) eluted. Evidently, the elution reagents and conditions must be compatible with the subsequent protein characterisation method which in this case was peptide mass fingerprinting by MALDI mass spectrometry. A large number of elution conditions were investigated with attention focussed on those that would minimise subsequent manipulation steps and be compatible with MALDI mass spectrometry. The applicant discovered that formic acid was particularly effective in eluting protein from a wide range of antibodies and was sufficiently volatile that it could be readily removed from the eluted protein samples prior to MALDI mass spectrometry. Thus, in this example immobilised antibody was incubated with a protein mixture (0.5-1.0mg; soya milk proteins) containing the protein antigen (BSA, 0.015-1.0mg) for 2h at room temperature. The wells were washed with PBS (6 x 0.35ml) and bound protein eluted by incubation with 20-100µl of 0.5% formic acid for 10 minutes at room temperature. The elution reagent was then removed by evaporating the samples to dryness under vacuum in a rotary evaporator.

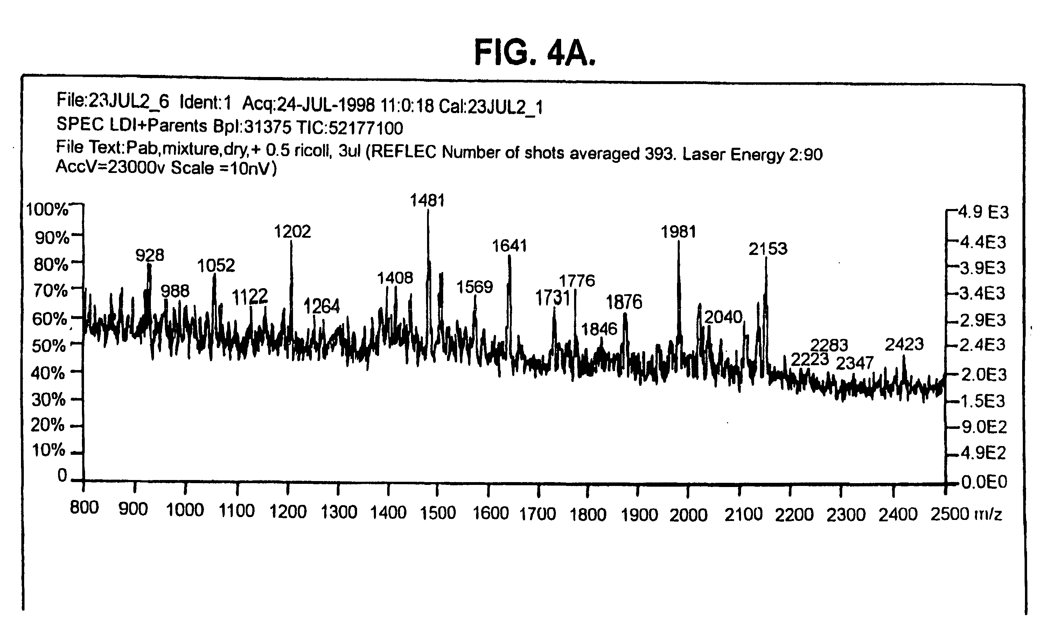

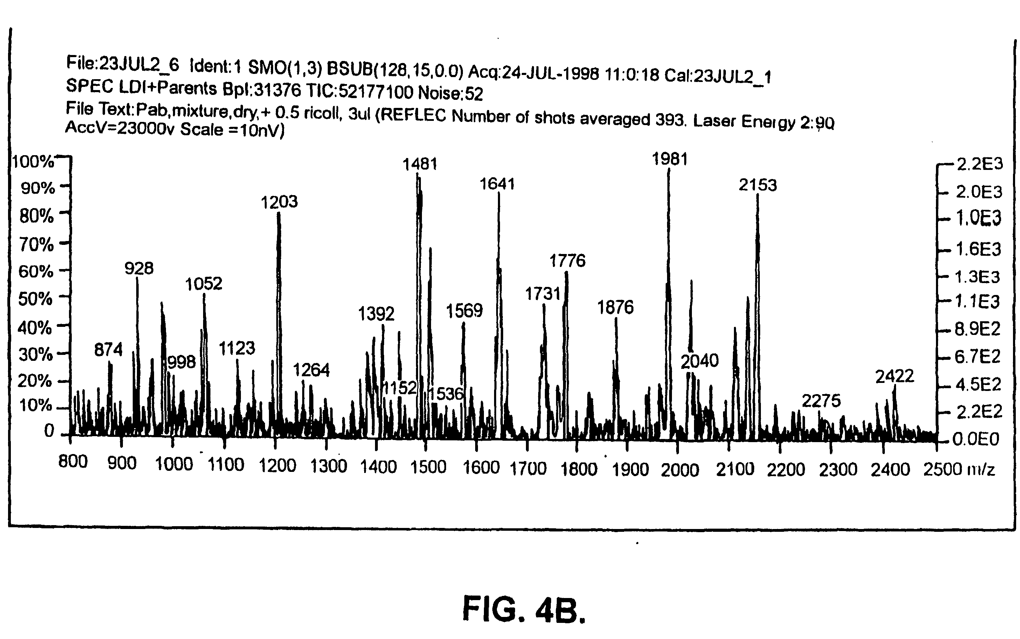

Eluted proteins were subjected to peptide mass fingerprinting by standard protocols (see Courchesne, P.L. and Patterson, S.D. (1999) 2-D Proteome Analysis Protocols [Link Ed.], *Methods in Molecular Biology* 112, 487-511. Humana Press). Thus, protein samples were subjected to enzymatic digestion (37°C, 18 hours) in 25mM ammonium bicarbonate, pH 7.8 containing 50ng of trypsin. Protein-doped crystals were prepared using α-cyano-4-hydroxycinnamic acid (HCCA) as a matrix. A saturated HCCA matrix solution (10mg/ml) was prepared in 70% MeCN/0.1 % TFA and any undissolved matrix removed by centrifugation. An aliquot (0.3-0.5µl) of sample was mixed with an equal volume of matrix solution on the target and allowed to air dry. In some cases it proved efficacious to remove impurities by washing the crystals by the addition of 5µl of ice cold 0.1% TFA which was aspirated after a few seconds. Washing steps were repeated up to 3 times. Samples were subjected to MALDI-mass spectrometry on a Lasermat 2000 (Thermo Bioanalysis) or TOFSPEC 2E (Micromass) and a representative mass spectrum shown in Figures 4A and 4B.

The predominant peptide masses from the spectrum shown in Figures 4A and 4B were selected 'blind' (after subtraction of peaks shown to originate from residual non-specific protein binding by use of an appropriate control - i.e. proteins recovered from wells in which no antibody had been immobilised) and used to search protein sequence databases. The peptide masses obtained in this way resulted in the identification of 3 matching proteins with BSA being identified as the 1^{st} ranking protein well above the other two ranked proteins.

### Example 5

### Inverse screening a monoclonal antibody against a target protein - bovine serum albumin

The inverse screening method was used to screen a commercially available monoclonal antibody to bovine serum albumin (BSA) against a mixture of proteins (containing BSA). Monoclonal anti-BSA antibody (5-80µg; Chemicon) in phosphate-buffered saline (PBS) was immobilised in the wells of 96 well filtration plates that incorporated a high protein binding hydrophobic PVDF membrane (Millipore MultiScreen® Immobilon™-P filtration plates) by incubating the pre-wetted membranes with the antibody containing solution for 2h at room temperature. The membranes were pre-wetted according to manufacturers instructions; thus to each well was added 50µl of 50% ethanol and the membranes incubated for I min at room temperature and after vacuum aspirating the wells each well was washed twice with 200µl of PBS. After antibody binding the wells were washed extensively with PBS (6 x 0.35ml). It is clear that different conditions for the immobilisation of antibody may be used and many have been examined in detail. These other methods include use of different buffers, different ... incubation times and temperatures, different methods for removal of remaining antibody solutions (vacuum aspiration through membrane or direct removal) all of which are known to those of skill in the art. The method described here proved optimal for the antibody used in this example. Other methods described where appropriate were optimal for the other examples cited.

The extent to which antibody binding had been effective was examined by several methods. Most routinely it was examined by determining the protein concentration (Bradford assay) of the antibody solution after it had been incubated with the wells. Under the conditions described greater than 95% of the antibody was immobilised when antibody solutions containing up to 40µg of antibody were added to each well.

Once antibody had been immobilised the remaining protein binding sites were blocked by incubation with 0.5% ficoll in PBS for 2h at room temperature. The wells were then washed extensively with PBS (6 x 0.35ml), incubated with elution reagent (0.5% formic acid) and washed again with PBS (6 x 0.35ml). Again, a number of different blocking conditions may be used and have been examined and these are obvious to those of skill in the art. One method found to be effective involved combining the ficoll block described above with dry blocking as recommended for the manufacturer for treatment of Immobilon™-P membranes when used in standard Western blotting applications. In this approach, the wells were dried by carefully removing all remaining solution and incubating in a desiccated environment at 37°C for 2h. The wells were then treated with 0.5% ficoll in PBS for 2h at room temperature, washed, treated with elution reagent and washed again as described above.

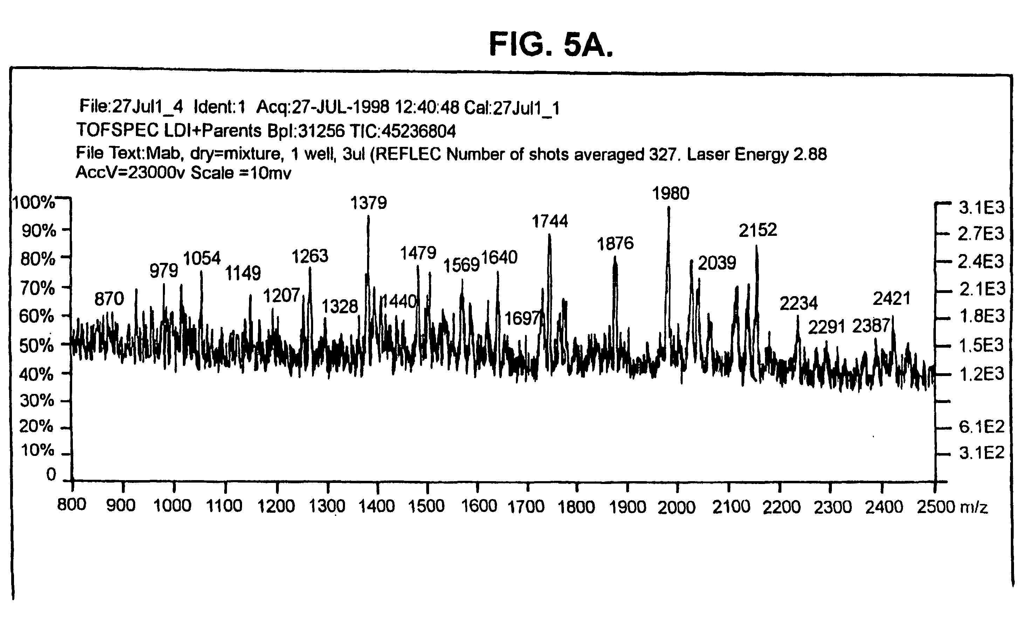

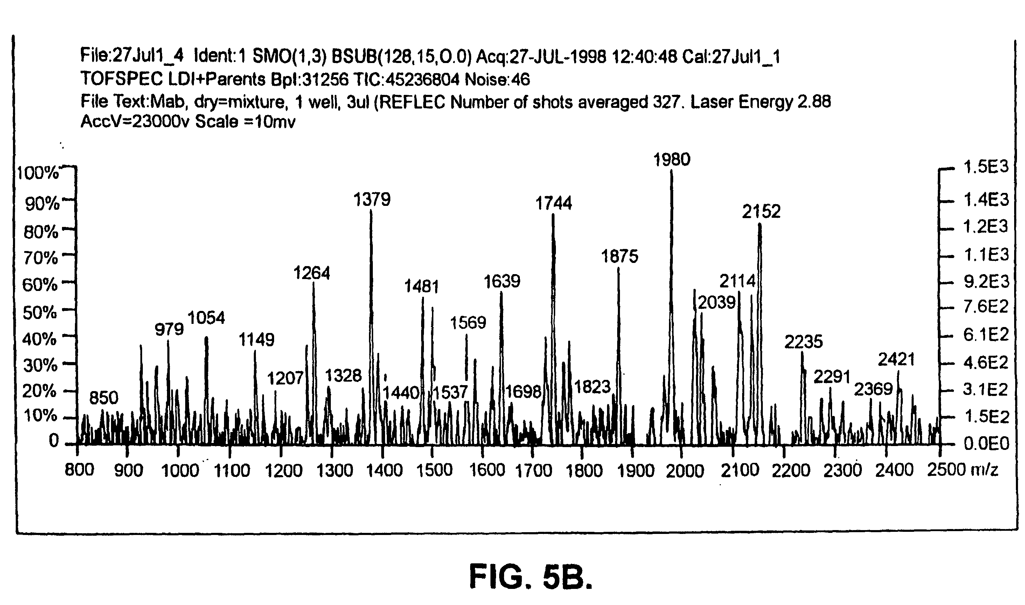

The immobilised antibody was then incubated with a protein mixture (0.5-1.0mg; soya milk proteins) containing the protein antigen (BSA, 0.015-1.0mg) for 2h at room temperature. The wells were washed with PBS (6 x 0.35ml) and bound protein eluted by incubation with 20-100µl elution reagent (0.5% formic acid) for 10 minutes at room temperature. The elution reagent was then removed and evaporated to dryness under vacuum in a rotary evaporator. Eluted proteins were subjected to peptide mass fingerprinting by standard protocols (see Courchesne, P.L. and Patterson, S.D. (1999) 2-D Proteome Analysis Protocols [Link Ed.], *Methods in Molecular Biology* 112, 487-511. Humana Press). Thus, protein samples were subjected to enzymatic digestion (37°C, 18 hours) in 25mM ammonium bicarbonate, pH 7.8 containing 50ng of trypsin. Protein-doped crystals were prepared using α-cyano-4-hydroxycinnamic acid (HCCA) as a matrix. A saturated HCCA matrix solution (10mg/ml) was prepared in 70% MeCN 0.1% TFA and any undissolved matrix removed by centrifugation. An aliquot (0.3-0.5µl) of sample was mixed with an equal volume of matrix solution on the target and allowed to air dry. In some cases it proved efficacious to remove impurities by washing the crystals by the addition of 5µl of ice cold 0.1 % TFA which was aspirated after a few seconds. Washing steps were repeated up to 3 times. Samples were subjected to MALDI-mass spectrometry on a Lasermat 2000 (Thermo Bioanalysis) or TOFSPEC 2E (Micromass) and a representative mass spectrum shown in Figures 5A and 5B.

The predominant peptide masses from the spectrum shown in Figure 5A and 5B were selected 'blind' (after subtraction of peaks shown to originate from residual non-specific protein binding by use of an appropriate control - proteins recovered from wells in which no antibody had been immobilised) and used to search protein sequence databases using publicly available software (for example see http://www.mann.embl-heidelberg.de/Services/PeptideSearch). Again BSA was identified as the 1^{st} ranking protein.

### Example 6

### Generation and inverse screening a monoclonal antibody against a target protein - bovine serum albumin

To generate monoclonal antibodies to bovine serum albumin (BSA) mice were immunised with the protein by standard methods (see Antibodies, a Laboratory Manual, E. Harlow and D. Lane (Eds.)). Thus, prior to immunisation a pre-bleed was taken from each mouse which was then injected subcutaneously at 2 sites with a 1:1 mixture of protein and Freund's incomplete adjuvant (100µl total). Twenty one days later tail bleeds were taken and each mouse injected subcutaneously at 2 sites with a further 100µl of a 1:1 mixture of protein and PBS. A further twenty one days later tail bleeds were again taken and each mouse injected subcutaneously at 2 sites with a further 100µl of a 1:1 mixture of protein and PBS. After a further 3 days some mice were splenectomised and a terminal bleed obtained. The splenocytes were extracted from the spleens of suitable mice, fused with myeloma cell line Sp2/0-Ag14 and cultured under selection pressure until the culture supernatants were ready for screening. Prior to screening by the inverse screening process hybridomas were screened by a conventional antibody capture ELISA (see Antibodies, a Laboratory Manual, E. Harlow and D. Lane (Eds.)). Thus, BSA was bound to the wells of 96 well plates by incubation with 0.5-10µg protein/well by standard procedures. Wells were blocked by incubation with 20% soya milk in PBS for 2 hours at room temperature and then washed with PBS before 100µl of hybridoma culture supernatant was applied to each well for 2 hours at room temperature. The wells were washed with PBS before a hybridoma screening anti-mouse Ig-HRP conjugate (Boehringer Mannheim) diluted in 20% soya milk was applied for 2 hours at room temperature. The wells were again washed with PBS before positive wells were visualised by the application of ABTS. It is important to emphasise that this pre-screening step was undertaken for convenience to identify positive hybridomas and hence to avoid unnecessary inverse screening of negative hybridomas. However, with sufficient time and effort or within a high-throughput environment such a pre-screening process is entirely unnecessary.

Wells containing actively growing hybridomas were cloned by limiting dilution and transferred to a serum-free medium. After an appropriate incubation period aliquots of culture supernatant containing antibody were subjected to inverse screening against a mixture of proteins (soya milk proteins containing BSA) as detailed in the previous examples. In this way peptide mass fingerprinting was used to identify those hybridoma clones producing monoclonal antibody to BSA. In some cases the efficacy of the antibody immobilisation and subsequent recovery of BSA from the protein mixture for analysis was improved by precipitation of the antibody from the tissue culture supernatant (using TCA and standard methods) prior to immobilisation. It is also noteworthy that the commercially available serum-free media for the maintenance and propagation of hybridomas were not compatible with direct recovery and immobilisation of antibody from the tissue culture supernatants. This seems most likely to arise from the presence of surfactants within such media preparations that attenuate antibody binding to Immobilon™-P. The invention includes provision for manipulation of the antibodies prior to immobilisation.

### Example 7

### Generation and inverse screening of monoclonal antibodies against yeast (S. cerevisiae) proteins

To generate monoclonal antibodies to *S. cerevisiae* proteins, mice were immunised with the protein by standard methods (see Antibodies, a Laboratory Manual, E. Harlow and D. Lane (Eds.)). Thus, prior to immunisation a pre-bleed was taken from each mouse which was then injected subcutaneously at 2 sites with a 1:1 mixture of protein and Freund's complete adjuvant (100µl total). Twenty one days later tail bleeds were taken and each mouse injected subcutaneously at 2 sites with a further 100µl of a 1:1 mixture of protein and PBS. A further twenty one days later tail bleeds were again taken and each mouse injected subcutaneously at 2 sites with a further 100µl of a 1:1 mixture of protein and PBS. To establish whether each animal was producing antibodies to a number of different proteins the serum from the various bleeds were used to Western blot yeast proteins separated by 1-DE SDS-polyacrylamide gel electrophoresis and transferred to nitrocellulose by standard procedures (see Antibodies, a Laboratory Manual, E. Harlow and D. Lane (Eds.)). The blots were blocked by incubation with 20% soya milk in PBS at 4°C overnight and then incubated with sera from day 0, 21 and 42 bleeds diluted 1:200 - 1:500 in 20% soya milk. Antibody binding to yeast was detected by incubation with anti-mouse Ig conjugated to horse radish peroxidase (Sigma) and the immunoblots developed by enhanced chemiluminescence (ECL, Amersham). The individual animals showed different patterns of antibody reactivity and were producing antibodies to a significant number of proteins (note 1-DE Western blotting will necessary underestimate the potential antibody repertoire of the animals). After a further 3 days some mice were splenectomised and a terminal bleed obtained. The splenocytes were extracted from the spleens and fused with myeloma cell line Sp2/0-Ag14 and cultured under selection pressure until the culture supernatants were ready for screening. Prior to screening by the inverse screening process hybridomas were screened by a conventional antibody capture ELISA (see Antibodies, a Laboratory Manual, E. Harlow and D. Lane (Eds.)). Thus, yeast proteins were bound to the wells of 96 well plates by incubation with 0.5-10µg protein/well by standard procedures. Wells were blocked by incubation with 20% soya milk in PBS for 2 hours at room temperature and then washed with PBS before 100µl of hybridoma culture supernatant was applied to each well for 2 hours at room temperature. The wells were washed with PBS before a hybridoma screening anti-mouse Ig-HRP conjugate (Boehringer-Mannheim) diluted in 20% soya milk was applied for 2 hours at room temperature. Wells containing actively growing hybridomas were cloned by limiting dilution and transferred to a serum-free medium. After an appropriate incubation period aliquots of culture supernatant containing antibody were subjected to inverse screening against a mixture of proteins (yeast proteins) as detailed in the previous examples. In this way peptide mass fingerprinting was and is being used to identify those hybridoma clones producing monoclonal antibodies to yeast proteins. It is noteworthy, that in this example no prior experimental determination of the peptide mass fingerprint of individual yeast proteins is necessary as the complete genome sequence of *S. cerevisiae* is known and hence the peptide mass fingerprints obtained from the inverse screening process can be compared directly with the theoretical peptide mass fingerprints for all *S. cerevisiae* proteins.

It is important to emphasise that both pre-screening steps (Western blotting and subsequently ELISA) were undertaken for convenience to confirm the potential value of each animal for the production of multiple antibodies and to identify positive hybridomas respectively and hence to avoid unnecessary inverse screening of negative hybridomas. However, with sufficient time and effort or within a high-throughput environment such a pre-screening processes would be entirely unnecessary.

## Claims

1. A method of selecting and/or identifying one or more protein affinity ligand's that bind to one or more proteins of interest comprising the steps of:
(A) obtaining a real or theoretical peptide mass fingerprint or other mass spectrometry based characterization or other protein characterization of the one or more proteins by either:
i. subjecting the one or more proteins to peptide mass fingerprinting or other mass spectrometry based characterization or other protein characterization; or
ii. predicting the peptide mass fingerprint or other mass spectrometry based characterization or other protein characterization from known data;
(B) utilizing the one or more proteins either individually or as a mixture to:
i. generate one or more antibodies thereto by immunisation; and/or
ii. select, using a single or multiple rounds of binding, one or more protein affinity ligands thereto;
(C) screening the one or more antibodies generated in step (B)(i) and/or the one or more protein affinity ligands selected in step (B)(ii) by:
(i) adding the one or more proteins individually or as a mixture of proteins to the one or more antibodies generated in step (B)(i) or the one or more protein affinity ligands selected in step (B)(i), each antibody or protein affinity ligand being used individually, and
(ii) after removing any proteins which have not bound, eluting the at least one protein that has bound;
(D) subjecting the at least one eluted protein to peptide mass fingerprinting and/or other mass spectrometry based characterization and/or other protein characterization; and
(E) by comparing the peptide mass fingerprints or other mass spectrometry based characterization or other protein characterization obtained in steps (A) and (D) selecting and/or identifying the at least one protein affinity ligand that binds to the one or more proteins of interest.

2. A method as claimed in claim 1 wherein the one or more proteins of interest are resolved by 2D electrophoresis.

3. A method as claimed in claims 1 or 2 wherein between steps (B) and (C) the antibodies obtained in step (B)(i) are cloned.

4. A method as claimed in any of the preceding claims wherein the one or more proteins of interest are present in a mixture of proteins.

5. A method as claimed in any of the preceding claims wherein the method is a shotgun method for selecting and identifying protein affinity ligands to a plurality of proteins.

6. A method as claimed in any of the preceding claims wherein the other mass spectrometry based characterization includes acquisition of sequence tag data.

7. A method as claimed in any of the preceding claims wherein the antibodies generated in step (B)(i) are immobilised on a support comprising nitrocellulose or PVDF.

8. A method as claimed in claim 7 wherein the support upon which the antibodies are immobilised are treated with oligosaccharide or polyvinylpyrrolidine solution to block any remaining binding sites.

9. A method as claimed in claim 8 wherein oligosaccharide is ficoll.

10. A method as claimed in any of claims 7 to 9 wherein the eluting agent is a volatile reagent.

11. A method as claimed in claim 10 wherein the volatile reagent is formic acid.

12. A method as claimed in any one or more of the previous claims wherein the one or more antibodies is monoclonal.

13. A method of generating an antibody library comprising the steps of:
(a) resolving a complex protein mixture and subjecting the resolved protein(s) to peptide mass finger printing to obtain a peptide mass profile; or
(b) obtaining a theoretical peptide mass profile for a protein which is sought;
(c) utilising the or another complex protein mixture to generate one or more monoclonal antibodies thereto;
(d) adding the or the other complex protein mixture to the one or more monoclonal antibodies generated in Step (c) to select those proteins which bind the one or more monoclonal antibodies, and subjecting the selected protein(s) to peptide mass fingerprinting to obtain a peptide mass profile;
(e) comparing the peptide mass profiles obtained in steps(a or b) and (d); and
(f) identifying the monoclonal antibodies of potential interest for a monoclonal antibody library.

14. A process for selecting desired members of an affinity ligand library comprising the steps of:
(a) resolving a complex protein mixture and subjecting the resolved protein(s) to peptide mass finger printing to obtain a peptide mass profile; or
(b) obtaining a theoretical peptide mass profile for a protein which is sought;
(c) utilising one or more of the resolved proteins to select one or more affinity ligands from a library;
(d) adding the or another complex protein mixture to the one or more affinity ligands generated in step (c) to select those proteins which bind the one or more affinity ligands, and subjecting the selected protein(s) to peptide mass fingerprinting to obtain a peptide mass profile;
(e) comparing the peptide mass profiles obtained in steps (a or b) and (d); and
(f) selecting one or more affinity ligands of interest.

## Patentansprüche

1. Verfahren zur Auswahl und/oder Identifizierung von einem oder mehr Protein-Affinitätsliganden, die an ein oder mehr Protein(e) von Interesse binden, umfassend die Schritte von:
(A) Erhalt eines echten oder theoretischen Peptid-Massenfingerprints oder einer anderen auf der Massenspektrometrie basierenden Charakterisierung oder einer anderen Proteincharakterisierung des einen Proteins oder von mehr Proteinen durch entweder:
i. Aussetzen des einen Proteins oder von mehr Proteinen dem Peptid-Massenfingerprinting oder einer anderen auf der Massenspektroskopie basierenden Charakterisierung oder einer anderen Proteincharakterisierung; oder
ii. Vorhersagen des Peptid-Massenfingerprints oder einer anderen auf der Massenspektrometrie basierenden Charakterisierung oder einer anderen Proteincharakterisierung aus bekannten Daten;
(B) Verwendung des einen Proteins oder von mehr Proteinen entweder individuell oder als ein Gemisch zum:
i. Generieren von einem oder mehr Antikörper(n) dazu durch Immunisierung; und/oder
ii. Auswählen, unter Verwendung von einer einzelnen oder mehrfachen Bindungsrunde(n), von einem oder mehr Protein-Affinitätsliganden dazu;
(C) Screening des in Schritt (B)(i) generierten einen Antikörpers oder von mehr Antikörpern und/oder des in Schritt (B)(ii) ausgewählten einen oder von mehr Protein-Affinitätsliganden durch:
(i) Zufügen des einen Proteins oder von mehr Proteinen, individuell oder als ein Gemisch aus Proteinen zum in Schritt (B)(i) generierten einen oder von mehr Antikörper(n), oder dem in Schritt (B)(i) ausgewählten einen oder von mehr Protein-Affinitätsliganden, wobei jeder Antikörper oder Protein-Affinitätsligand individuell verwendet wird, und
(ii) nach Entfernung jeglicher Proteine, die nicht gebunden wurden, Eluieren des mindestens einen Proteins, das gebunden wurde;
(D) Aussetzen des mindestens einen eluierten Proteins dem Peptid-Massenfingerprinting und/oder einer anderen auf der Massenspektrometrie basierenden Charakterisierung und/oder einer anderen Proteincharakterisierung; und
(E) durch Vergleich der in Schritten (A) und (D) erhaltenen Peptid-Massenfingerprints oder einer anderen auf der Massenspektroskopie basierenden Charakterisierung oder einer anderen Proteincharakterisierung, wobei der mindestens eine Protein-Affinitätsligand, der an ein oder mehr Protein(e) von Interesse bindet, ausgewählt und/oder identifiziert wird.

2. Verfahren nach Anspruch 1, worin das eine oder mehr Protein(e) von Interesse durch 2D-Elektrophorese aufgetrennt wird/werden.

3. Verfahren nach Anspruch 1 oder 2, worin zwischen Schritten (B) und (C) die in Schritt (B)(i) erhaltenen Antikörper kloniert werden.

4. Verfahren nach einem der vorangehenden Ansprüche, worin das eine oder mehr Protein(e) von Interesse in einem Gemisch von Proteinen anwesend ist/sind.

5. Verfahren nach einem der vorangehenden Ansprüche, worin das Verfahren ein Shotgun-Verfahren zur Auswahl und Identifizierung von Protein-Affinitätsliganden an einer Vielzahl von Proteinen darstellt.

6. Verfahren nach einem der vorangehenden Ansprüche, worin die andere auf der Massenspektrometrie basierende Charakterisierung die Erfassung von "Sequence-Tag"-Daten einschließt.

7. Verfahren nach einem der vorangehenden Ansprüche, worin die in Schritt (B)(i) generierten Antikörper auf einem Träger, umfassend Nitrocellulose oder PVDF, immobilisiert sind.

8. Verfahren nach Anspruch 7, worin der Träger, auf dem die Antikörper immobilisiert sind, mit Oligosaccharid- oder Polyvinylpyrrolidon-Lösung zum Blockieren jeglicher verbleibender Bindungsstellen behandelt werden.

9. Verfahren nach Anspruch 8, worin das Oligosaccharid Ficoll darstellt.

10. Verfahren nach einem der Ansprüche 7 bis 9, worin das Elutionsmittel ein flüchtiges Reagens darstellt.

11. Verfahren nach Anspruch 10, worin das flüchtige Reagens Ameisensäure darstellt.

12. Verfahren nach einem oder mehr der vorangehenden Ansprüche, worin der eine oder mehr Antikörper monoklonal ist/sind.

13. Verfahren zur Generierung einer Antikörperbibliothek, umfassend die Schritte von:
(a) Auftrennung eines komplexen Proteingemischs und Aussetzen des aufgetrennten Proteins/der aufgetrennten Proteine dem Peptid-Massenfingerprinting zum Erhalt eines Profils von der Peptidmasse; oder
(b) Erhalt eines theoretischen Profils von der Peptidmasse für ein Protein, das gesucht wird;
(c) Verwendung des oder eines anderen komplexen Proteingemischs zum Generieren von einem oder mehr monoklonalen Antikörper(n) dazu;
(d) Zufügen des oder des anderen komplexen Proteingemischs an den in Schritt (c) generierten einen oder von mehr monoklonalen Antikörper(n) zur Auswahl der Proteine, die den einen oder mehr monoklonale(n) Antikörper binden und Aussetzen des/der ausgewählten Proteins/Proteine dem Peptid-Massenfingerprinting zum Erhalt eines Profils von der Peptidmasse;
(e) Vergleich der in Schritten (a oder b) und (d) erhaltenen Profile von der Peptidmasse; und
(f) Identifizierung der monoklonalen Antikörper von potenziellem Interesse für eine monoklonale Antikörperbibliothek.

14. Verfahren zur Auswahl gewünschter Glieder einer Affinitätsligandenbibliothek, umfassend die Schritte von:
(a) Auftrennung eines komplexen Proteingemischs und Aussetzen des/der aufgetrennten Proteins/Proteine dem Peptid-Massenfingerprinting zum Erhalt eines Profils von der Peptidmasse; oder
(b) Erhalt eines theoretischen Profils von der Peptidmasse für ein Protein, das gesucht wird;
(c) Nutzung von einem oder mehr der aufgetrennten Proteine zur Auswahl von einem oder mehr Affinitätsliganden aus einer Bibliothek;
(d) Zufügen des oder eines anderen komplexen Proteingemischs zum in Schritt (c) generierten einen oder von mehr Affinitätsliganden zur Auswahl der Proteine, die den einen oder mehr Affinitätsliganden binden und Aussetzen des/der ausgewählten Proteins/Proteine dem Peptid-Massenfingerprinting zum Erhalt eines Profils von der Peptidmasse;
(e) Vergleich der in Schritten (a oder b) und (d) erhaltenen Profile von der Peptidmasse; und
(f) Auswahl eines oder von mehr Affinitätsliganden von Interesse.

## Revendications

1. Procédé de sélection et/ou d'identification d'un ou plusieurs ligands à affinité protéique qui se lie à une ou plusieurs protéines d'intérêt comprenant les étapes consistant à:
(A) obtenir une cartographie peptidique de masse réelle ou théorique ou une autre caractérisation basée sur la spectrométrie de masse ou une autre caractérisation protéique de l'une ou plusieurs protéines soit :
i. en soumettant la seule ou les plusieurs protéines à une cartographie peptidique de masse ou à une autre caractérisation basée sur la spectrométrie de masse ou à une autre caractérisation protéique ; soit
ii. en prédisant la cartographie peptidique de masse ou une autre caractérisation basée sur la spectrométrie de masse ou une autre caractérisation protéique obtenue à partir de données connues ;
(B) utiliser la seule ou les plusieurs protéines soit individuellement soit sous la forme d'un mélange pour :
i. produire un ou plusieurs anticorps contre cette(ces) protéine(s) par immunisation ; et/ou
ii. sélectionner, en utilisant une seule ou de multiples étapes de fixation, un ou plusieurs ligands à affinité protéique pour cette(ces) protéine(s) ;
(C) cribler le seul ou les plusieurs anticorps générés dans l'étape (B)(i) et/ou le seul ou les plusieurs ligands à affinité protéique sélectionnés dans l'étape (B)(ii) :
(i) en ajoutant la seule ou les plusieurs protéines individuellement ou sous la forme d'un mélange de protéines au seul ou aux plusieurs anticorps générés dans l'étape (B)(i) ou au seul ou aux plusieurs ligands à affinité protéique sélectionnés dans l'étape (B)(i), chaque anticorps ou ligand à affinité protéique étant utilisé individuellement, et
(ii) après suppression des protéines qui ne se sont pas liées, en éluant l'au moins seule protéine qui s'est liée ;
(D) soumettre l'au moins seule protéine éluée à une cartographie peptidique de masse et/ou à une autre caractérisation basée sur la spectrométrie de masse et/ou à une autre caractérisation protéique ; et
(E) en comparant les cartographies peptidiques de masse ou l'autre caractérisation basée sur la spectrométrie de masse ou l'autre caractérisation protéique obtenues dans les étapes (A) et (D), sélectionner et/ou identifier l'au moins seul ligand à affinité protéique qui se lie à la seule ou aux plusieurs protéines d'intérêt.

2. Procédé selon la revendication 1 dans lequel la seule ou les plusieurs protéines d'intérêt sont analysées par électrophorèse 2D.

3. Procédé selon la revendication 1 ou 2 dans lequel entre les étapes (B) et (C) les anticorps obtenus dans l'étape (B)(i) sont clonés.

4. Procédé selon l'une quelconque des revendications précédentes dans lequel la seule ou les plusieurs protéines d'intérêt sont présentes dans un mélange de protéines.

5. Procédé selon l'une quelconque des revendications précédentes dans lequel le procédé est un procédé de séquençage aléatoire afin de sélectionner et d'identifier les ligands à affinité protéique pour une pluralité de protéines.

6. Procédé selon l'une quelconque des revendications précédentes dans lequel l'autre caractérisation basée sur la spectrométrie de masse comprend l'acquisition des données relatives aux marqueurs de séquences.

7. Procédé selon l'une quelconque des revendications précédentes dans lequel les anticorps générés dans l'étape (B)(i) sont immobilisés sur un support comprenant de la nitrocellulose ou du PVDF.

8. Procédé selon la revendication 7 dans lequel le support sur lequel les anticorps sont immobilisés sont traités avec une solution contenant un oligosaccharide ou une solution de polyvinylpyrrolidine pour bloquer tout site de liaison restant.

9. Procédé selon la revendication 8 dans lequel l'oligosaccharide est le ficoll.

10. Procédé selon l'une quelconque des revendications 7 à 9 dans lequel l'agent d'élution est un réactif volatil.

11. Procédé selon la revendication 10 dans lequel le réactif volatil est l'acide formique.

12. Procédé selon l'une quelconque ou plusieurs des revendications précédentes dans lequel le seul ou les plusieurs anticorps sont monoclonaux

13. Procédé de création d'une librairie d'anticorps comprenant les étapes consistant à :
(a) analyser un mélange de protéines complexes et soumettre la (les) protéine(s) analysée(s) à une cartographie peptidique de masse pour obtenir un profil peptidique de masse ; ou
(b) obtenir un profil peptidique de masse théorique pour une protéine qui est recherchée ;
(c) utiliser le mélange ou un autre mélange de protéines complexes pour produire un ou plusieurs anticorps monoclonaux contre les protéines du mélange ;
(d) ajouter le mélange ou l'autre mélange de protéines complexes au seul ou aux plusieurs anticorps monoclonaux produits dans l'étape (c) pour sélectionner ces protéines qui se lient au seul ou aux plusieurs anticorps monoclonaux, et soumettre la (les) protéine(s) sélectionnée(s) à une cartographie peptidique de masse pour obtenir un profil peptidique de masse ;
(e) comparer les profils peptidiques de masse obtenus dans les étapes (a ou b) et (d) ; et
(f) identifier les anticorps monoclonaux d'intérêt potentiel pour une librairie d'anticorps monoclonaux.

14. Procédé pour sélectionner les membres souhaités d'une librairie de ligands à affinité comprenant les étapes consistant à :
(a) analyser un mélange de protéines complexes et soumettre la (les) protéine(s) analysée(s) à une cartographie peptidique de masse pour obtenir un profil peptidique de masse ; ou
(b) obtenir un profil peptidique de masse théorique pour une protéine qui est recherchée ;
(c) utiliser une ou plusieurs des protéines analysées pour sélectionner un ou plusieurs ligands à affinité à partir d'une librairie ;
(d) ajouter le mélange ou un autre mélange de protéines complexes au seul ou aux plusieurs ligands d'affinité produits dans l'étape (c) pour sélectionner ces protéines qui se lient au seul ou aux plusieurs ligands d'affinité, et soumettre la (les) protéine(s) sélectionnée(s) à une cartographie peptidique de masse pour obtenir un profil peptidique de masse ;
(e) comparer les profils peptidiques de masse obtenus dans les étapes (a ou b) et (d) ; et
(f) sélectionner un ou plusieurs ligands à affinité d'intérêt.
